# EUROPÄISCHE PATENTSCHRIFT

(11) **EP 3 377 167 B1**
(45) Veröffentlichungstag und Bekanntmachung des Hinweises auf die Patenterteilung: **29.01.2020**
(21) Anmeldenummer: 16797473.2
(22) Anmeldetag: 03.11.2016
(51) Int. Cl.: A61M 39/22, F16K 27/06, F16K 11/085

(54) **MEHRWEGHAHN MIT MEHRFACHHALTERING**
MULTI-WAY VALVE COMPRISING A MULTIPLE RETENTION RING
ROBINET À VOIES MULTIPLES POURVU D'UNE BAGUE DE RETENUE MULTIPLE

(30) Priorität: 17.11.2015 DE 102015119899
(43) Veröffentlichungstag der Anmeldung: 26.09.2018
(73) Patentinhaber: Hopf, Hans-Jürgen, 90513 Zirndorf (DE)
(72) Erfinder: HOPF, Michael, 90513 Zirndorf (DE); HOPF, Alexander, 90491 Nürnberg (DE); KASSAI, Norbert, 90522 Oberasbach (DE); HOPF, Hans-Jürgen, 90513 Zirndorf (DE)
(74) Vertreter: Wittmann, Ernst-Ulrich
(86) Internationale Anmeldenummer: PCT/EP2016/076586
(87) Internationale Veröffentlichungsnummer: WO 2017/084890

(56) Entgegenhaltungen:
- DE-A1-102009 026 172
- US-A1- 2005 245 899
- US-A1- 2005 252 560
- US-B1- 6 237 628

## Beschreibung

Die vorliegende Erfindung betrifft einen Mehrweghahn, insbesondere 2-,3- und 4-Weghähne wie sie insbesondere in der Medizin eingesetzt werden.

Mehrweghähne insbesondere 2-,3- oder4-Weghähne sind im Stand der Technik bekannt und werden insbesondere in der Medizin und Medizintechnik eingesetzt. Hierbei werden sie insbesondere im Infusionsbereich, dem Bereich der künstlichen Ernährung, bei derTransfusion und insbesondere für die Zu- bzw. Überleitung von verschiedenen Durchflussmedien und als sogenanntes "Injektions-Equipment" für die medizinische und pharmazeutische Ausrüstung verwendet. Die Mehrweghähne werden ferner auch in medizinischen Systemen verwendet, welche unter anderem aus mehreren Komponenten bestehen. Solche Systeme umfassen unter anderem Schwerkraftinfusionen, Pumpen oder Pumpüberleitungssysteme, Sondennahrungssysteme, Injektionen, Kombinationen hiervon und dergleichen.

Ein Mehrweghahn kann ferner durch die Kombination mit mehreren Mehrweghähnen zu einer sog. Mehrwegehahnbank oder "Manifold" (Mehrfachverteiler) montiert werden.

Bei der Bereitstellung von Mehrweghähnen ist von Bedeutung, dass insbesondere eine vorgegebene Dichtheit, eine leichte Bedienungsanwendung und eine Gratfreiheit der Bauteile gegeben ist. Insbesondere ist hierbei zu beachten, dass Mehrweghähne zur Zuführung unterschiedlicher Medien verwendet werden, welche unter anderem sehr unterschiedliche Viskositäten aufweisen.

Im Stand der Technik ist beispielsweise die US 6,237,628 B1 bekannt, welche die Konstruktion eines Wasserablassventiles zum Ablassen von angesammeltem Wasser aus dem Inneren eines Kraftstofffilters, der mit einem Wasserabscheidevermögen ausgeführt ist, offenbart.

Die DE 10 2009 026 172 A1 offenbart einen Mehrweghahn für den Einsatz in der Medizin, der die Kombination der Merkmale des Oberbegriffs von Anspruch 1 zeigt. Dabei weist das Grundgehäuse des Mehrweghahn ein um eine zentrale Achse drehbewegliches Stellglied auf.

Die US 2005/0245899 A1 offenbart einen Anschluss für Flüssigkeitsansaug- und Flüssigkeitszufuhranwendungen, ohne dass zusätzliche Adapter oder Gummihülse zur Verbindung einer Vakuumröhre notwendig sind.

Die US 2005/0252560 A1 offenbart einen Absperrventil welches an einer Vielzahl an Leitungen verbunden werden kann.

In den im Stand der Technik bekannten Mehrweghähnen ist es unter anderem von Nachteil, dass diese in verschiedenen Anwendungsfällen Schwächen aufweisen, die unter anderem die Dichtheit, die Bedienerfreundlichkeit und Probleme beim Durchfluss verschieden viskoser Medien sind.

Aufgabe der vorliegenden Erfindung ist es, die im Stand der Technik bekannten Nachteile wenigstens teilweise zu überwinden. Die vorstehende Aufgabe wird durch einen erfindungsgemäßen Mehrweghahn gemäß Anspruch 1 gelöst. Bevorzugte Ausgestaltungsformen des Mehrweghahns sind Gegenstand der entsprechenden Unteransprüche.

Der erfindungsgemäße Mehrweghahn zum Einsatz in der Medizin und/oder Medizintechnik weist ein wenigstens abschnittsweise von einem Medium durchströmbares Grundgehäuse und ein in dem Grundgehäuse um eine zentrale Achse drehbeweglich aufgenommenes Stellglied auf. Der erfindungsgemäße Mehrweghahn ist dadurch gekennzeichnet, dass erAnschlussstellen für den Zulauf und den Ablauf des Mediums aufweist.

Des Weiteren bildet das Grundgehäuse des erfindungsgemäßen Mehrweghahns eine Stellgliedaufnahme, welche dadurch gekennzeichnet ist, dass sie einen konzentrisch zur zentralen Achse des Grundgehäuses angeordneten Zapfen ausweist. Das Stellglied des erfindungsgemäßen Mehrweghahns weist ein Bedienelement, sowie einen im Wesentlichen zylindrischen Abschnitt auf, der den Zapfen der Stellgliedaufnahme des Grundgehäuses wenigstens abschnittsweise aufnimmt und darüber hinaus wenigstens eine Durchflussöffnung zur Fluidverbindung wenigstens zweier Anschlussstellen aufweist.

Der Zapfen des Grundgehäuses des erfindungsgemäßen Mehrweghahns ist so ausgebildet, dass er wenigstens im Bereich einer Anschlussstelle des Grundgehäuses ein Gegenlagerfür das Stellglied aufweist beziehungsweise bereitstellt, welches zusammen mit dem Stellglied eine Dichtfläche im Bereich wenigstens einer Anschlussstelle bildet. Der erfindungsgemäße Mehrweghahn ist dadurch gekennzeichnet, dass der Mehrweghahn Ausformungen und Aussparungen aufweist, welche jeweils zueinander korrespondierend angeordnet sind und welche zusammen eine Fügeverbindung zwischen Stellglied und Stellgliedaufnahme bilden und wenigstens zwei Fügeverbindungen vorgesehen sind, wobei jeweils wenigstens eine Ausformung und/oder Aussparung radial umlaufend an der Innenseite des Grundgehäuses des Stellglieds bzw. radial umlaufend an der Außenseite des zylindrischen Abschnitts der Stellgliedaufnahme angeordnet ist.

Unter Medium im Sinne der vorliegenden Erfindung werden alle Arten von Fluiden, insbesondere Flüssigkeiten, wie beispielsweise wässrige Flüssigkeiten, Blut, Ernährungsbrei, etc. und auch Gase verstanden.

Unter dem Grundgehäuse im Sinne der vorliegenden Erfindung wird ein Bauteil des Mehrweghahns, welcher das Gehäuse mit den Anschlussstellen und der Stellgliedaufnahme aufweist, verstanden, wobei unter letzterer der Bereich des Grundgehäuses verstanden wird, welcher zur Aufnahme des Stellglieds dient.

Unter Stellglied im Sinne der vorliegenden Erfindung wird ein, wenigstens eine Strömungsöffnung abschließendes, sogenanntes "Küken" verstanden. Das Bedienelement im Sinne der vorliegenden Erfindung ist ein mit dem Stellglied fest verbundenes Element, wie zum Beispiel ein Hahn, mit dem sich das Stellglied um die zentrale Achse drehen lässt. Dieser kann einen oder mehrere, üblicherweise zwei, drei oder vier Stellhebel aufweisen.

Unter Gegenlager im Sinne der vorliegenden Erfindung wird ein Bereich derStellgliedaufnahme verstanden, der den Druck auf das Stellglied, insbesondere bei geschlossenem Zustand aufnimmt, bzw. einer Verformung des Stellglieds unter dem Druck des Mediums entgegen wirkt.

Die Modifikation des Stellglieds und der Stellgliedaufnahme durch die erfindungsgemäße Erhöhung der Anzahl an zueinander korrespondierend angeordneten Ausformungen und Aussparungen, welche zusammen wenigstens zwei Fügeverbindungen zwischen Stellglied und Stellgliedaufnahme bilden, führt zu einer höheren Abzugsfestigkeit zwischen Stellglied und Stellgliedaufnahme. Dadurch wird sichergestellt, dass der Hahn nicht durch das eingesetzte, insbesondere viskose Medium, aus seiner definierten, eingestellten Position verschoben werden kann und damit gleichzeitig ein Leckschlagen des eingesetzten, insbesondere viskosen Mediums ganz oder nahezu vermieden.

Gemäß einer bevorzugten Ausführungsform des erfindungsgemäßen Mehrweghahns, sind die Aussparungen und die Ausformungen jeweils paarweise in einer Folge von zwei Aussparungen und anschließend von zwei Ausformungen oder *vice versa* in einer Folge von zwei Ausformungen und anschließend von zwei Aussparungen (d.h. im Muster 2+2) an dem Stellglied bzw. an der Stellgliedaufnahme jeweils zueinander korrespondierend angeordnet. Gemäß einer weiteren, bevorzugten Ausführungsform des erfindungsgemäßen Mehrweghahns, sind die Aussparungen paarweise und die Ausformungen einzeln in einer Folge von zwei Aussparungen und anschließend einer Ausformung oder in einer Folge von einer Ausformung und anschließend zwei Aussparungen oder in einer Folge von einer Ausformung und anschließend von zwei Aussparungen und nachfolgend einer Ausformung (d.h. im Muster 2+1, 1+2 oder 1+2+1) an dem Stellglied bzw. an der Stellgliedaufnahme jeweils zueinander korrespondierend angeordnet. Gemäß einer weiteren bevorzugten Ausführungsform sind die Ausformungen paarweise und die Aussparungen einzeln in einer Folge von zwei Ausformungen und anschließend einer Aussparung oder in einer Folge von einer Aussparung und anschließend zwei Ausformungen oder in einer Folge von einer Aussparung und anschließend von zwei Ausformungen und nachfolgend einer Aussparung (d.h. im Muster 2+1, 1+2 oder 1+2+1) an dem Stellglied bzw. an der Stellgliedaufnahme jeweils zueinander korrespondierend angeordnet. In einer weiteren, bevorzugten Ausführungsform des erfindungsgemäßen Mehrweghahns, sind die Aussparungen in Dreier-Gruppen und die Ausformungen einzeln in einer Folge von drei Aussparungen und anschließend einer Ausformung oder in einer Folge von einer Ausformung und anschließend von drei Aussparungen (d.h. im Muster3+1 oder 1+3) an dem Stellglied bzw. an der Stellgliedaufnahme jeweils zueinander korrespondierend angeordnet. In einer weiteren, bevorzugten Ausführungsform des erfindungsgemäßen Mehrweghahns, sind die Ausformungen in Dreier-Gruppen und die Aussparungen einzeln in einer Folge von drei Ausformungen und anschließend einer Aussparung oder in einer Folge von einer Aussparung und anschließend von drei Ausformungen (d.h. im Muster3+1 oder 1+3) an dem Stellglied bzw. an der Stellgliedaufnahme jeweils zueinander korrespondierend angeordnet. Durch die Anordnung der Aussparungen und Ausformungen der vorgenannten bevorzugten Ausführungsformen im Sinne einer Labyrinthdichtung wird neben einer verbesserten mechanischen Fixierung auch eine bessere Dichtheit der angeordneten Bauteile erreicht.

Gemäß einer weiteren, bevorzugten Ausführungsform des erfindungsgemäßen Mehrweghahns sind alle Aussparungen an dem Stellglied bzw. alle Ausformungen an der Stellgliedaufnahme angeordnet, oder gemäß einer weiteren, bevorzugten Ausführungsform sind die Aussparungen und die Ausformungen alternierend an dem Stellglied bzw. an der Stellgliedaufnahme jeweils zueinander korrespondierend angeordnet.

Erfindungsgemäß ist bei der Stellgliedaufnahme der Durchmesser der Aussparungen bzw. der Durchmesser derAusformungen jeweils graduell kleiner in Richtung des Inneren der Stellgliedaufnahme, dergestalt, dass die innerste Aussparung bzw. die innerste Ausformung den kleinsten Durchmesser der vorhandenen Aussparungen bzw. Ausformungen aufweist und der Durchmesser jeder zusätzlich vorhandenen Aussparung bzw. Ausformung dann in Richtung der Oberfläche der Stellgliedaufnahme graduell ansteigt, so dass die am äußersten Rand gelegene Aussparung bzw. Ausformung, den größten Durchmesser der vorhandenen Aussparungen bzw. Ausformungen zukommt. Gleichsam ist entsprechend beim Stellglied der Durchmesser der Aussparungen bzw. der Durchmesser der Ausformungen jeweils graduell kleiner in Richtung des zylindrischen Abschnitts des Stellglieds, dergestalt, dass die innerste Aussparung bzw. die innerste Ausformung den kleinsten Durchmesser der vorhandenen Aussparungen bzw. Ausformungen aufweist und der Durchmesser jeder zusätzlich vorhandenen Aussparung bzw. Ausformung dann in Richtung Bedienelement des Stellglieds graduell ansteigt, so dass die dem Bedienelement am nächsten gelegene Aussparung bzw. Ausformung, den größten Durchmesserdervorhandenen Aussparungen bzw. Ausformungen zukommt. Diese graduelle Ausführung ermöglicht, dass bei der Montage des erfindungsgemäßen Mehrweghahns bei dem Einsenken des Stellglieds in die Stellgliedaufnahme die Aussparungen und korrespondierenden Ausformungen der äußeren und inneren Fügeverbindungen gleichzeitig formschlüssig und nur in den vorgesehenen Fügeverbindungspositionen einrasten, d.h. ohne dass die inneren Aussparungen bzw. Ausformungen des Stellglieds zuvor bei einer anderen Ausformung bzw. Aussparung der Stellgliedaufnahme einrasten, bevor sie ihre endgültige Position innerhalb des Mehrweghahns eingenommen haben.
In einer weiteren Ausführungsform des erfindungsgemäßen Mehrweghahns, weist das Grundgehäuse zwei, drei oder vier Anschlussstellen auf.
Gemäß einer weiteren, besonders bevorzugten Ausführungsform ist das den Mehrweghahn durchströmende Medium eine Flüssigkeit, insbesondere ausgewählt aus einer Gruppe, welche Injektionslösungen, Infusionslösungen, Nährlösungen (inklusive Ernährungsbrei), Blut, Plasma sowie Kombinationen hieraus und dergleichen aufweist.

Gemäß einer weiteren, besonders bevorzugten Ausführungsform weist die den Mehrweghahn durchströmende Flüssigkeit eine Viskosität auf, welche 0,7 mPa·s⁻¹ und 10⁶ mPa·s⁻¹, bevorzugt zwischen 1 mPa·s⁻¹ und 10⁵ mPa·s⁻¹, und besonders bevorzugt bei etwa 10² mPa·s⁻¹ liegt.

Gemäß einer weiteren Ausführungsform des erfindungsgemäßen Mehrweghahns, ist der Zapfen des Grundgehäuses wenigstens abschnittsweise konisch ausgeführt. In einerweiteren, besonders bevorzugten Ausführungsform sind konisch und nicht konische und/oder konische Abschnitte mit verschiedenen Konuswinkel in stufiger Abfolge ausgeführt. Der Konuswinkel gemäß der vorliegenden Situation wird als Abweichung von der zentralen Achse verstanden; insbesondere werden positive und negative Winkel unter Konuswinkel verstanden. In einer weiteren, besonders bevorzugten Ausführungsform weist der Zapfen eine alternierende Folge positiver und negativer Konuswinkel auf. Im einer weiteren bevorzugten Ausführungsform weist der Zapfen relativ zurzentralen Achse nach innen oder außen versetzte, insbesondere konische Abschnitte auf.

Gemäß einer weiteren bevorzugten Ausführungsform ist die Innenseite des zylindrischen Abschnitts wenigstens abschnittsweise konisch ausgeführt und ist insbesondere an den konischen Verlauf des Zapfen im Wesentlichen angepasst.

Gemäß der vorliegenden Erfindung sind die Durchflussöffnungen des Stellglieds torförmig ausgeführt, insbesondere derartig ausgeführt, dass die Durchflussöffnung zum Ende des zylindrischen Abschnitts des Stellglieds offen ist. Ferner wird insbesondere die Durchflussöffnung vorzugsweise in Abhängigkeit des zu verwendenden Fluids in ihrer Größe angepasst, wobei ferner auch eine Kombination der Form des Gegenlagers wie beispielsweise konisch und rechteckig mit berücksichtigt wird.

Gemäß einer weiteren, besonders bevorzugten Ausführungsform des erfindungsgemäßen Mehrweghahns entspricht die Zahl der Durchflussöffnungen des Stellglieds der Zahl der Anschlussstellen am Grundgehäuse. So weißt zum Beispiel eine Ausführungsform mit zwei Anschlussstellen zwei Durchflussöffnungen auf. Gemäß einer weiteren bevorzugten Ausführungsform weist das Stellglied weniger Durchlassöffnungen auf, als Anschlussstellen am Grundgehäuse vorhanden sind, wobei wenigstens und insbesondere zwei Durchlassöffnungen bzw. als Tore ausgestaltete Durchlassöffnungen vorgesehen sind.

Gemäß einer weiteren, besonders bevorzugten Ausführungsform des Mehrweghahns gemäß der vorliegenden Erfindung weistjede Fügeverbindung einen radial umlaufenden Dichtabschnitt und/oder einen Positionierabschnitt auf. Insbesondere sind die wenigstens zwei Fügeverbindungen zwischen dem Stellglied und dem Grundgehäuse der Stellgliedaufnahme so gestaltet, dass durch die Verbindungen zwischen den beiden Teilen eine Dichtheit von bevorzugt größer als 3 bar, besonders bevorzugt größer als 8 bar und am meisten bevorzugt größer als 10 bar erreicht wird. Vorzugsweise wird eine Dichtheit im Bereich von größer etwa 3 bar bis größer etwa 8 bar erreicht.

Dies ermöglicht, dass der erfindungsgemäße Mehrweghahn auch für viskose Fluide wie beispielsweise Nahrungsbrei (z.B. für Sondennahrungssysteme), bei dessen Einsatz aufgrund der höheren Mediumsviskosität höhere Drücke auftreten, welche die Fügeverbindungen des Mehrweghahns angreifen, ohne Leckzuschlagen eingesetzt werden kann.

Gemäß einer weiteren bevorzugten Ausführungsform weisen die Ausformungen an dem Stellglied bzw. an der Stellgliedaufnahme eine teilrunde Form auf und die jeweils korrespondierenden Aussparungen an der Stellgliedaufnahme bzw. an dem Stellglied sind entsprechend zurformschlüssigen Aufnahme der korrespondierenden teilrunden Ausformung und Bildung der jeweiligen Fügeverbindung in ihrer Form angepasst.

Gemäß einer weiteren, besonders bevorzugten Ausführungsform des Mehrweghahns gemäß der vorliegenden Erfindung ist der zylindrische Abschnitt des Stellglieds wenigstens abschnittsweise aus Vollmaterial gebildet. Insbesondere ist der zylindrische Abschnitt des Stellglieds im Bereich der Fügeverbindung aus Vollmaterial gebildet. Dadurch wird gegenüber einer hohlzylindrischen Ausführung eine höhere Formstabilität und Materialsteifigkeit im betreffenden Abschnitt erreicht und damit eine verbesserte Abzugsfestigkeit zwischen dem Stellglied und der Stellgliedaufnahme ermöglicht, um sicherzustellen, dass ein Leckschlagen des eingesetzten, insbesondere viskosen Mediums ganz oder nahezu vermieden wird. Insbesondere wird damit eine Abzugsfestigkeit von größer als 80 N, bevorzugt größer 150 N, besonders bevorzugt größer als 200 N und am meisten bevorzugt größer als 250 N erreicht. Vorzugsweise wird eine Abzugsfestigkeit im Bereich von größer 150 N bis größer 250 N erreicht.

Gemäß einer weiteren bevorzugten Ausführungsform des erfindungsgemäßen Mehrweghahns wird wenigstens das Gehäuse und/oder das Stellglied wenigstens teilweise aus einem Material hergestellt, das aus einer Gruppe ausgewählt ist, welche neben Copolyester weitere Materialien wie zum Beispiel duro- und thermoplastische Kunststoff und insbesondere Polyphenylensulfid, Polypropylen, Poly-1-buten, Polyvinylchlorid, Polyvinylidenchlorid, Polymethyl-metaacrylat, Polyacrylnitril, Polystyrol, Polysulfon, Polyacetal, Polyvinylalkohol, Polyvinylacetat, lonomere, Fluorkunststoff, Polyethylen, Polyamid, insbesondere ein teilaromatisches Polyamid, Polycarbonat, Polyester, Copolyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurethan, Polyvinylchlorid, ungesättigtes Polyesterharz, antimikrobielle oder antiseptische Materialen wie beispielsweise hochporöses Silber, ionenfrei hergestelltes Silber, Silberverbindungen und insbesondere Microsilber, Metallionen freisetzende Verbindungen und Materialien, welche keine Weichmacher insbesondere kein Bisphenol A oder Phthalate enthalten, sowie Kombinationen hiervon und dergleichen umfasst.

Gemäß einerweiteren bevorzugten Ausführungsform ist der erfindungsgemäße Mehrweghahns dadurch gekennzeichnet, dass wenigstens die mit dem Fluid in Kontakt kommenden Oberflächen des Verbindungssystems, insbesondere die innenliegenden Oberflächen des Mehrweghahns wenigstens abschnittsweise aus einem Material hergestellt sind, welches antiseptische und/oder antimikrobielle Eigenschaften aufweist.

Solche antimikrobielle oder antiseptische Materialen sind beispielsweise Materialien wie hochporöses Silber, ionenfrei hergestelltes Silber, Silberverbindungen und insbesondere MicroSilber, Metallionen freisetzende Verbindungen, Kombinationen hiervon und dergleichen. Vorzugsweise werden diese im Bereich der entsprechenden Oberflächen angeordnet, wobei gemäß einer weiteren besonders bevorzugten Ausführungsform die entsprechenden Materialien auch in dem Kunststoff, aus welchem der Mehrwegehahn bzw. dessen Bestandteile hergestellt sind eingebunden sind. So kann insbesondere ein hochporöses Silber, welches vorzugsweise auch im Wesentlichen ionenfrei hergestellt ist, in den Kunststoff eingemischt werden, wobei hieraus dann die Komponenten des Mehrweghahns wenigstens abschnittsweise hergestellt werden. Alternativ liegt es auch im Sinn der vorliegenden Erfindung die Oberflächen mit einem entsprechenden Material bzw. Materialkombination zu beschichten.

Gemäß einer weiteren besonders bevorzugten Ausführungsform dervorliegenden Erfindung wird ein amorpher Copolyester zur Herstellung des Mehrweghahns für die Medizin und Medizintechnik und dessen Verwendung insbesondere für Infusions- oder Transfusionsschläuche, Mehrwegehähne, Mehrfachverteiler, Injektionsequipment wie Nadeln, Zugänge oder ähnliches und Kombinationen hiervon verwendet. Dabei ist das System dadurch gekennzeichnet, dass wenigstens die mit dem Fluid in Kontakt kommenden Oberflächen des Verbindungssystems wenigstens abschnittsweise aus einem amorphen Copolyester hergestellt werden.

Gemäß einer weiteren, besonders bevorzugten Ausführungsform werden wenigstens teilweise Materialien für die Herstellung des erfindungsgemäßen Verbindungssystems verwendet, welche keine Weichmacher, insbesondere keine Phthalate und kein Bisphenol A enthalten.

Die vorliegende Erfindung umfasst ferner auch die Verwendung des erfindungsgemäßen Mehrweghahns in der Medizin und Medizintechnik, insbesondere für die Zu- und Überleitung von verschiedenen Durchflussmedien, insbesondere für die Schwerkraftinfusion, für Pumpüberleitungssysteme, Sondennahrungssysteme, Injektionen sowie Kombinationen hiervon und dergleichen.

Die Erfindung wird nachfolgen anhand eines bevorzugten Ausführungsbeispiels erläutert, wobei darauf hingewiesen wird, dass durch dieses Beispiel Abwandlungen beziehungsweise Ergänzungen wie sie sich für den Fachmann unmittelbar ergeben mit umfasst sind. Darüber hinaus stellt dieses bevorzugte Ausführungsbeispiel keine Beschränkung der Erfindung in der Art dar, dass Abwandlungen und Ergänzungen im Umfang der vorliegenden Erfindung liegen.

Dabei zeigen:
Fig. 1 bis 3 eine Draufsicht und Seitenansicht auf eine besonders bevorzugte Ausführungsform eines Mehrweghahns;
Fig. 4 bis 6 eine Draufsicht auf unterschiedlich ausgestaltete Mehrweghähne;
Fig. 7 einen Querschnitt durch das Grundgehäuse eines erfindungsgemäßen Dreiweghahns;
Fig. 8 einen Querschnitt durch das Grundgehäuse eines erfindungsgemäßen Dreiweghahns aus Fig. 7 mit eingesenktem Stellglied;

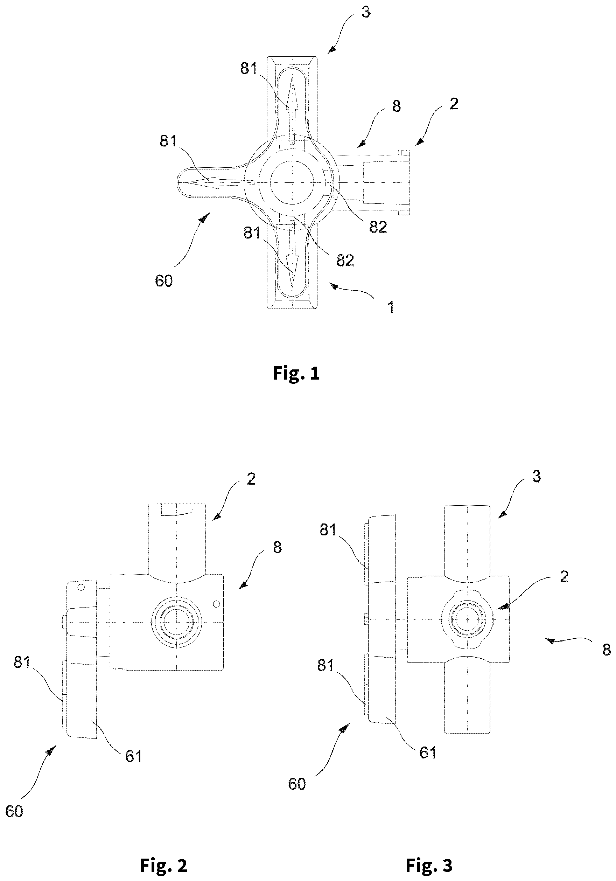

Die Figuren 1 bis 3 zeigen je eine Draufsicht in Figur 1 und je eine Seitenansicht in Figuren 2 und 3 einer Ausgestaltungsform eines erfindungsgemäßen Mehrweghahns. Figur 1 zeigt das Grundgehäuse 8 mit drei Anschlussstellen 1, 2, 3. Das Stellglied 60 weist ein Bedienelement 61 in Form eines Hahnes mit drei Stellhebeln auf, welches fest mit einem zylindrischen Abschnitt 62 (nicht dargestellt) verbunden ist. Der zylindrische Abschnitt 62 ist konzentrisch um eine zentrale Achse 6 (nicht dargestellt) angeordnet. An dem Bedienelement gegenüberliegendem Ende des zylindrischen Abschnitts 62 sind torförmige Durchlassöffnungen 82 (Tore) ausgebildet. Die hier dargestellte Ausführungsform zeigt dabei ein Stellglied 60, bei welchem im zylindrischen Abschnitt 62 nur zwei Durchgänge 82 (Tore) vorgesehen sind, die insbesondere zueinander mit einem Winkel von 90° angeordnet sind. Gemäß dieser Anordnung werden je nach Schaltung die Anschlüsse 1 und 2 oder 2 und 3 fluidverbunden. Eine Verbindung zwischen dem Anschluss 3 und 1 ist nicht möglich.

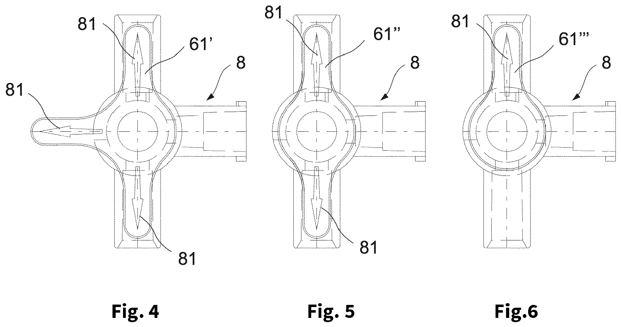

Die Figuren 4 bis 6 zeigen je eine Draufsicht von unterschiedlich ausgestalteten Mehrweghähnen, bei welchen insbesondere die Bedienelemente 61', 61", 61''' unterschiedlich ausgestaltet sind. So zeigt Figur4 ein dreiarmiges Bedienelement 61', Figur5 ein zweiarmiges Bedienelement 61" und Figur 6 ein einarmiges Bedienelement, wie es insbesondere in Kombination mit unterschiedlich ausgestalteten Grundgehäusen 8 verwendet werden kann. Die auf dem Bedienelement angebrachten Pfeile 81 dienen ferner der leichteren Orientierung beim Einsatz der Mehrweghähne in der Praxis, um schematisch die Flussrichtung des Fluids anzugeben.

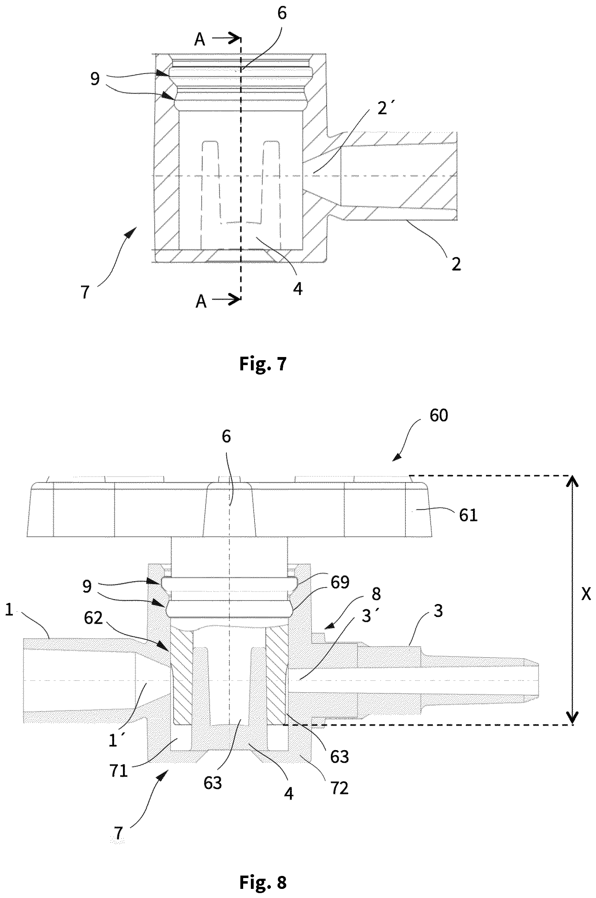

Das Ausführungsbeispiel gemäß Figur 7 zeigt einen Querschnitt des Grundgehäuses 8 (mit grobliniger Schraffur dargestellt) mit zwei radial um die zentrale Achse 6 umlaufende Aussparungen 9 auf der Innenseite der Stellgliedaufnahme 7, wobei jede Aussparung 9 jeweils so gestaltet ist, dass sie unterschiedliche Tiefen aufweist. In der Stellgliedaufnahme 7 ist um eine zentrale Achse 6 ein Zapfen 4 angeordnet. Die Anschlussstelle 2 ist mit der Stellgliedaufnahme verbunden, wobei die Durchlassöffnung 2' gebildet wird. Der Durchmesser der inneren Aussparung 9 hat in diesem Ausführungsbeispiel einen kleineren Durchmesser als die äußere Aussparung 9 und beide Aussparungen 9 weisen eine zurteilrunden Form der Ausformungen 69 des aufsteckbaren Stellglieds 60 (nicht gezeigt; vgl. aber Figur 8) angepasste Form auf, um einen Formschluss der Fügeverbindungen bei der Montage des erfindungsgemäßen Mehrweghahns und damit eine erhöhte Dichtheit des Mehrweghahns zu gewährleisten. Die unterbrochenen Linien, welche mit A-A beschriftet sind, markieren die Schnittlinie A-A für die Querschnitte in der Figur 8.

So zeigt Figur 8 die Verbindung des Grundgehäuses 8 (mit feinliniger Schraffur dargestellt) in Richtung der A-A-Schnittlinie mit dem Stellglied 60 entsprechend der Figur 7 als eine Ausführungsform des erfindungsgemäßen Mehrweghahns. Das Stellglied 60 ist bis zu einer vorbestimmten Einpresstiefe X in die Stellgliedaufnahme 7 des Grundgehäuses eingepresst. In dieser Position greifen die zwei teilrunden Ausformungen 69 des zylindrischen Abschnitts 62 des Stellglieds 60 formschlüssig in die beiden Aussparungen 9 an der Innenseite der Stellgliedaufnahme 7 des Grundgehäuses 8. Der zylindrische Abschnitt 62 (mit grobliniger Schraffur dargestellt) des Stellglieds 60 umfasst den um eine zentrale Achse 6 angeordnete Zapfen 4 der Stellgliedaufnahme 7 und bildet so ein Gegenlagerfür das Stellglied 60. Der Durchfluss des Mediums im geöffneten Zustand des Mehrweghahns erfolgt von einer der Anschlussstellen 1, 3 durch die zugehörige Durchlassöffnung des Grundgehäuses 1', 3' durch eine torförmige Durchlassöffnung 63 des Stellglieds 60 über einen Freiraum 71 zwischen dem Stellglied 60 und dem Boden 72 der Stellgliedaufnahme 7 hin zur einer anderen Durchlassöffnung 63 des Stellglieds und durch die jeweils andere Durchlassöffnung 1', 3' des Grundgehäuses aus derjeweils anderes Anschlussstelle 1, 3 am Grundgehäuses hinaus. Die Dichtheit im oberen Bereich wird durch eine definierte Einpresstiefe des Stellglieds 60 in den Fügerandes erreicht. Im Inneren des Grundgehäuses 8 wird die Dichtheit zu den entsprechenden Durchlassöffnungen 1, 2, 3 über das konische Gegenlager, gebildet durch den Zapfen 4, sichergestellt.

## Patentansprüche

1. Mehrweghahn für den Einsatz in der Medizin oder Medizintechnik, mit wenigstens einem abschnittsweise von einem Medium durchströmbaren Grundgehäuse (8) und einem hierin, um eine zentrale Achse (6) drehbeweglich aufgenommenen Stellglied (60), wobei
das Grundgehäuse (8) wenigstens zwei Anschlussstellen (1, 2, 3) für den Zu- und Abfluss des Mediums aufweist und eine Stellgliedaufnahme (7) bildet, welche einen konzentrisch zur zentralen Achse (6) angeordneten Zapfen (4) aufweist, und
das Stellglied (60) ein Bedienelement (61) und einen, im Wesentlichen zylindrischen Abschnitt (62) aufweist, welcher den Zapfen (4) der Stellgliedaufnahme (7) wenigstens abschnittsweise aufnimmt und darüber hinaus wenigstens eine Durchflussöffnung (63) zur Fluidverbindung wenigstens zweier Anschlussstellen (1, 2, 3) aufweist und
der Zapfen (4) der Stellgliedaufnahme (7) wenigstens ein Gegenlager für das Stellglied (60) zur Bildung einer Dichtfläche im Bereich wenigstens einer Anschlussstelle (1, 2, 3) aufweist
**dadurch gekennzeichnet, dass**
der Mehrweghahn Ausformungen (69) und Aussparungen (9) aufweist, welche jeweils zueinander korrespondierend angeordnet sind und welche zusammen eine Fügeverbindung zwischen Stellglied (60) und Stellgliedaufnahme (7) bilden und wenigstens zwei Fügeverbindungen vorgesehen sind,
wobei jeweils wenigstens eine Ausformung (69) und/oder Aussparung (9) radial umlaufend an der Außenseite des zylindrischen Abschnitts (62) des Stellglieds (60) bzw. radial umlaufend an der Innenseite der Stellgliedaufnahme (7) des Grundgehäuses (8) angeordnet ist und wobei alle Aussparungen (9) an dem Stellglied (60) bzw. alle Ausformungen (69) an der Stellgliedaufnahme (7) angeordnet sind, oder die Aussparungen (9) und Ausformungen (69) alternierend an dem Stellglied (60) bzw. an der Stellgliedaufnahme (7) angeordnet sind, und
der Durchmesser der Aussparungen (9) bzw. der Durchmesser der Ausformungen (69) jeweils graduell kleiner in Richtung des Inneren der Stellgliedaufnahme (7) ist,
so dass bei der Montage bei dem Einsenken des Stellglieds (60) in die Stellgliedaufnahme (7) die Aussparungen (9) und korrespondierenden Ausformungen (69) der äußeren und inneren Fügeverbindungen bei einer vorbestimmten Einpresstiefe (X) gleichzeitig formschlüssig und nur in den vorgesehenen Fügeverbindungspositionen einrasten.

2. Mehrweghahn gemäß einem der Ansprüche 1 oder 2, **dadurch gekennzeichnet, dass** das Grundgehäuse (8) zwei, drei oder vier Anschlussstellen (1, 2, 3) aufweist.

3. Mehrweghahn gemäß einem der vorstehenden Ansprüche, **dadurch gekennzeichnet, dass**
das Medium insbesondere eine Flüssigkeit ist, die aus einer Gruppe ausgewählt wird, welche Injektionslösungen, Infusionslösungen, Nährlösungen, Blut, Plasma, Gase, Kombinationen hieraus und dergleichen aufweist.

4. Mehrweghahn gemäß Anspruch 4, **dadurch gekennzeichnet, dass**
die Flüssigkeit eine Viskosität aufweist, welche zwischen 0,7 mPa·s⁻¹ und 10⁶ mPa ·s⁻¹ insbesondere zwischen 1 mPa ·s⁻¹ und 10⁵ mPa·s⁻¹, und besonders bevorzugt bei etwa 10² mPa ·s⁻¹ liegt.

5. Mehrweghahn gemäß einem der vorstehenden Ansprüche, **dadurch gekennzeichnet, dass**
der Zapfen (4) wenigstens abschnittsweise konisch, vorzugsweise in stufiger Abfolge und/oder insbesondere mit unterschiedlichen Konuswinkeln ausgeführt ist.

6. Mehrweghahn gemäß einem der vorstehenden Ansprüche, **dadurch gekennzeichnet, dass**
die Innenseite des zylindrischen Abschnitts (62) des Stellglieds (60) wenigstens abschnittsweise konisch ausgeführt ist und insbesondere dem konischen Verlauf des Zapfens (4) im Wesentlichen entspricht.

7. Mehrweghahn gemäß einem der vorstehenden Ansprüche, **dadurch gekennzeichnet, dass**
die Durchflussöffnungen (63) des Stellglieds (60) als Tore ausgeführt sind, welche insbesondere zum Ende des zylindrischen Abschnitts (62) des Stellglieds offen sind.

8. Mehrweghahn gemäß einem der vorstehenden Ansprüche, **dadurch gekennzeichnet, dass**
die Zahl der Durchflussöffnungen (63) des Stellglieds (60) der Zahl der Anschlussstellen (1, 2, 3) im Grundgehäuse (8) entspricht.

9. Mehrweghahn gemäß einem der vorstehenden Ansprüche, **dadurch gekennzeichnet, dass**
jede Fügeverbindung einen radial umlaufenden Dichtabschnitt und/oder Positionierabschnitt aufweist.

10. Mehrweghahn gemäß einem der vorstehenden Ansprüche, **dadurch gekennzeichnet, dass**
die wenigstens zwei vorhandenen Fügeverbindungen zwischen dem Stellglied (60) und dem Grundgehäuse (8) der Stellgliedaufnahme (7) so gestaltet sind, dass durch die Verbindungen zwischen den beiden Teilen eine Dichtheit von bevorzugt größer als 3 bar, besonders bevorzugt größer als 8 bar und am meisten bevorzugt größer als 10 bar erreicht wird.

11. Mehrweghahn gemäß einem der vorstehenden Ansprüche, **dadurch gekennzeichnet, dass**
der zylindrische Abschnitt (62) des Stellglieds (60) wenigstens abschnittsweise aus Vollmaterial gebildet ist, insbesondere im Bereich der Fügeverbindung aus Vollmaterial gebildet ist.

12. Mehrweghahn gemäß einem der vorstehenden Ansprüche, **dadurch gekennzeichnet, dass**
wenigstens das Grundgehäuse (8) und/oder das Stellglied (60) wenigstens teilweise aus einem Material hergestellt sind, das aus einer Gruppe ausgewählt ist, welche neben Copolyester, insbesodere amorphe Copolyester, weitere Materialien wie zum Beispiel duro- und thermoplastische Kunststoff und insbesondere Polyphenylensulfid, Polypropylen, Poly-1-buten, Polyvinylchlorid, Polyvinylidenchlorid, Polymethyl-metaacrylat, Polyacrylnitril, Polystyrol, Polysulfon, Polyacetal, Polyvinylalkohol, Polyvinylacetat, lonomere, Fluorkunststoff, Polyethylen, Polyamid, insbesondere ein teilaromatisches Polyamid, Polycarbonat, Polyester, Copolyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurethan, Polyvinylchlorid, ungesättigtes Polyesterharz, antimikrobielle oder antiseptische Materialen wie beispielsweise hochporöses Silber, ionenfrei hergestelltes Silber, Silberverbindungen und insbesondere Microsilber, Metallionen freisetzende Verbindungen und Materialien, welche keine Weichmacher, insbesondere kein Bisphenol A oder Phthalate enthalten, sowie Kombinationen hiervon und dergleichen umfasst.

## Claims

1. A multi-way valve for use in medicine or medical technology, comprising at least one main housing (8) through parts of which a medium can flow, and an actuator (60) which is accommodated therein in such a way as to be rotatable about a central axis (6), wherein
the main housing (8) has at least two connection points (1, 2, 3) for admitting and discharging the medium and forms an actuator receptacle (7) which has a peg (4) that is arranged concentrically with the central axis (6), and
the actuator (60) has a control element (61) and a substantially cylindrical portion (62) which accommodates at least a portion of the peg (4) of the actuator receptacle (7) and which additionally has at least one throughflow opening (63) for fluidically connecting at least two connection points (1, 2, 3)
and
the peg (4) of the actuator receptacle (7) has at least one abutment for the actuator (60) such that a sealing surface is formed in the region of at least one connection point (1, 2, 3),
**characterised in that**
the multi-way valve has protrusions (69) and recesses (9), which are arranged so as to correspond with each other and which together form a joint connection between the actuator (60) and actuator receptacle (7), with at least two joint connections bring provided, wherein at least one protrusion (69) and/or recess (9) is arranged radially circumferentially on the outer side of the cylindrical portion (62) of the actuator (60) or radially circumferentially on the inner side of the actuator receptacle (7) of the main housing (8), and wherein all recesses (9) are arranged on the actuator (60) and/or all protrusions (69) are arranged on the actuator receptacle (7), or the recesses (9) and protrusions (69) are arranged in an alternating manner on the actuator (60) and/or the actuator receptacle (7), and
the diameter of the recesses (9) and/or the diameter of the protrusions (69) becomes gradually smaller in the direction of the interior of the actuator receptacle (7), so that at the time of assembly when lowering the actuator (60) into the actuator receptacle (7), the recesses (9) and corresponding protrusions (69) of the outer and inner joint connections simultaneously engage interlockingly and only in the envisaged joint connection positions at a predetermined press-in depth (X).

2. The multi-way valve according to any one of claims 1 or 2, **characterised in that** the main housing (8) has two, three or four connection points (1, 2, 3).

3. The multi-way valve according to any one of the preceding claims, **characterised in that** the medium is in particular a fluid which is selected from a group which comprises solutions for injection, solutions for infusion, nutritional solutions, blood, plasma, gases, and combinations thereof.

4. The multi-way valve according to claim 4 **characterised in that** the fluid has a viscosity which is between 0.7 mPa (s⁻¹) and 10⁶ mPa (s⁻¹), in particular between 1 mPa (s⁻¹) and 10⁵ mPa (s⁻¹), and particularly preferably approximately 10² mPa (s⁻¹).

5. The multi-way valve according to any one of the preceding claims, **characterised in that** the peg (4) is at least partially conical, preferably with a stepped sequence and/or in particular is formed with different cone angles.

6. The multi-way valve according to any one of the preceding claims, **characterised in that** the inner side of the cylindrical portion (62) of the actuator (60) is at least partially conical and in particular corresponds substantially to the conical course of the peg (4).

7. The multi-way valve according to any one of the preceding claims, **characterised in that** the throughflow openings (63) of the actuator (60) are designed as gates, which are open in particular towards the end of the cylindrical portion (62) of the actuator.

8. The multi-way valve according to any one of the preceding claims, **characterised in that** the number of throughflow openings (63) of the actuator (60) corresponds to the number of connection points (1, 2, 3) in the main housing (8).

9. The multi-way valve according to any one of the preceding claims, **characterised in that** each joint connection has a radially circumferential sealing portion and/or positioning portion.

10. The multi-way valve according to any one of the preceding claims, **characterised in that** the at least two joint connections between the actuator (60) and the main housing (8) of the actuator receptacle (7) are designed such that a sealing of preferably greater than 3 bar, particularly preferably greater than 8 bar, and most preferably greater than 10 bar is achieved by the connections between the two parts.

11. The multi-way valve according to any one of the preceding claims, **characterised in that** the cylindrical portion (62) of the actuator (60) at least in part is made of solid material, in particular is formed from solid material in the region of the joint connection.

12. The multi-way valve according to any one of the preceding claims, **characterised in that** at least the main housing (8) and/or the actuator (60) are at least in part made of a material selected from a group which in addition to copolyester, in particular amorphous copolyester, also includes further materials, for example thermosets and thermoplastics and in particular polyphenyl sulphide, polypropylene, poly-1-butene, polyvinylchloride, polyvinylidene chloride, polymethyl metaacrylate, polyacrylonitrile, polystyrene, polysulfone, polyacetal, polyvinyl alcohol, polyvinyl acetate, ionomers, fluorine plastics, polyethylene, polyamide, in particular a partially aromatic polyamide, polycarbonate, polyester, copolyester, polyphenylene oxide, polysulfone, polyvinyl acetal, polyurethane and chlorinated polyether, cellulose nitrate, cellulose acetate, cellulose ether, phenol resin, urea resin, thiourea resin, melamine resin, alkyl resin, allyl resin, silicone, polyimide, polybenzimidazole, epoxy resin, casein plastic, crosslinked polyurethane, polyvinyl chloride, unsaturated polyester resin, antimicrobial or antiseptic materials, for example highly porous silver, ion-free silver, silver compounds and in particular micro-silver, compounds releasing metal ions, and materials containing no softening agent, in particular not bisphenol A or phthalates, as well as combinations thereof and the like.

## Revendications

1. Robinet à voies multiples pour l'emploi dans la médecine ou dans la technique médicale, avec au moins une boîte de base (8) traversable par section par un fluide, et un composant de réglage (60) logé dedans de manière mobile par rotation autour d'un axe central (6),
la boîte de base (8) présentant au moins deux points de raccordement (1, 2, 3) pour l'arrivée et l'écoulement du fluide et constituant un réceptacle de composant de réglage (7), qui présente un pivot (4) disposé concentriquement par rapport à l'axe central (6), et
le composant de réglage (60) présentant un élément de commande (61) et une section cylindrique pour l'essentiel (62), qui reçoit le pivot (4) du réceptacle de composant de réglage (7) au moins par section et présentant par ailleurs au moins un orifice d'écoulement (63) en vue de la liaison fluide d'au moins deux points de raccordement (1, 2, 3), et
le pivot (4) du réceptacle de composant de réglage (7) présentant au moins un palier-support pour le composant de réglage (60) en vue de la constitution d'une surface d'étanchéité dans la zone d'au moins un point de raccordement (1, 2, 3),
**caractérisé en ce que**
le robinet à voies multiples présente des façonnages (69) et des évidements (9) qui sont disposés respectivement l'un à l'autre de manière correspondante et qui constituent ensemble une liaison de jonction entre le composant de réglage (60) et le réceptacle de composant de réglage (7), et au moins deux liaisons de jonction sont prévues,
respectivement au moins un façonnage (69) et/ou évidement (9) est disposé radialement en périphérie sur le côté extérieur de la section cylindrique (62) du composant de réglage (60) et/ou radialement en périphérie sur le côté intérieur du réceptacle de composant de réglage (7) de la boîte de base (8), et
tous les évidements (9) étant disposés sur le composant de réglage (60) et/ou tous les façonnages (69) sur le réceptacle de composant de réglage (7), ou les évidements (9) et les façonnages (69) étant disposés en alternance sur le composant de réglage (60) et/ou sur le réceptacle de composant de réglage (7), et
le diamètre des évidements (9) et/ou le diamètre des façonnages (69) étant respectivement graduellement inférieur dans la direction de l'intérieur du réceptacle de composant de réglage (7),
de sorte que lors du montage, à la plongée du composant de réglage (60) dans le réceptacle de composant de réglage (7), les évidements (9) et les façonnages (69) correspondants des liaisons de jonction extérieures et intérieure s'enclenchent simultanément de manière mécanique et uniquement dans les positions de liaisons de jonction prévues à une profondeur d'enfoncement prédéterminée (X).

2. Robinet à voies multiples selon une quelconque des revendications 1 ou 2, **caractérisé en ce que** la boîte de base (8) présente deux, trois ou quatre points de raccordement (1, 2, 3).

3. Robinet à voies multiples selon une quelconque des revendications précédentes, **caractérisé en ce que**
le fluide est notamment un liquide qui est sélectionné à partir d'un groupe qui comprend des solutions d'injection, des solutions de perfusion, des bouillons de culture, du sang, du plasma, des gaz, des combinaisons de ceux-ci et similaires.

4. Robinet à voies multiples selon la revendication 4, **caractérisé en ce que** le liquide présente une viscosité qui se trouve entre 0,7 mPa ·s⁻¹ et 10⁶ mPa ·s⁻¹, notamment entre 1 mPa ·s⁻¹ et 10⁵ mPa ·s⁻¹, et de manière particulièrement favorisée à environ 10² mPa ·s⁻¹

5. Robinet à voies multiples selon une quelconque des revendications précédentes, **caractérisé en ce que**
le pivot (4) est réalisé au moins par section coniquement, de préférence en séquence par paliers et/ou notamment avec des angles coniques différents.

6. Robinet à voies multiples selon une quelconque des revendications précédentes, **caractérisé en ce que**
le côté intérieur de la section cylindrique (62) du composant de réglage (60) est réalisé coniquement au moins par sections et correspond pour l'essentiel notamment au profil conique du pivot (4).

7. Robinet à voies multiples selon une quelconque des revendications précédentes, **caractérisé en ce que**
les orifices d'écoulement (63) du composant de réglage (60) sont réalisés comme des portes, qui sont notamment ouvertes vers l'extrémité de la section cylindrique (62) du composant de réglage.

8. Robinet à voies multiples selon une quelconque des revendications précédentes, **caractérisé en ce que**
le nombre des orifices d'écoulement (63) du composant de réglage (60) correspond au nombre des points de raccordement (1, 2, 3) dans la boîte de base (8).

9. Robinet à voies multiples selon une quelconque des revendications précédentes, **caractérisé en ce que**
chaque liaison de jonction présente une section de colmatage et/ou de positionnement s'étendant radialement en périphérie.

10. Robinet à voies multiples selon une quelconque des revendications précédentes, **caractérisé en ce que**
au moins les deux liaisons de jonction présentes entre le composant de réglage (60) et la boîte de base (8) du réceptacle de composant de réglage (7) sont agencées de sorte que, par les liaisons entre les deux parties, une étanchéité de préférence supérieure à 3 bars, de préférence particulière supérieure à 8 bars, et de préférence maximale à 10 bars, est atteinte.

11. Robinet à voies multiples selon une quelconque des revendications précédentes, **caractérisé en ce que**
la section cylindrique (62) du composant de réglage (60) est constituée au moins par section en matériau massif, notamment est constituée en matériau massif dans la zone de la liaison de jonction.

12. Robinet à voies multiples selon une quelconque des revendications précédentes, **caractérisé en ce que**
au moins la boîte de base (8) et/ou le composant de réglage (60) sont fabriqués au moins partiellement en un matériau qui est sélectionné à partir d'un groupe, qui, outre le copolyester, comprend notamment du copolyester amorphe, d'autres matériaux comme par exemple les matières thermodurcissables et thermoplastiques, et notamment le polysulfure de phénylène, le polypropylène, le poly-1-butène, le chlorure de polyvinyle, le chlorure de polyvinylidène, le polyméthacrylate de méthyle, le polyacrylonitrile, le polystyrène, le polysulfone, le polyacétal, l'alcool polyvinylique, le polyacétate de vinyle, les ionomères, le plastique fluoré, le polyéthylène, le polyamide, notamment un polyamide partiellement aromatique, le polycarbonate, le polyester, le copolyester, l'oxyde de polyphénylène, le polysulfone, le polyacétal de vinyle, le polyuréthane, et le polyéther chloré, le nitrate de cellulose, l'acétate de cellulose, l'éther de cellulose, la résine phénolique, la résine d'urée, la résine de thio-urée, la résine de mélamine, la résine d'alcoyle, la résine allylique, la silicone, le polyimide, le polybenzimidazole, la résine époxy, le plastique à la caséine, le polyuréthane réticulé, le chlorure de polyvinyle, la résine de polyester insaturée, des matériaux antimicrobiens ou antiseptiques comme par exemple l'argent hautement poreux, l'argent fabriqué sans ions, des combinaisons d'argent et notamment le micro-argent, des combinaisons et matériaux dégageant des ions métalliques, qui ne contiennent pas de plastifiants, notamment pas de bisphénol-A ou de phthalates, ainsi que des combinaisons de ceux-ci et similaires.
